# EUROPEAN PATENT SPECIFICATION

(11) **EP 3 478 822 B1**
(45) Date of publication and mention of the grant of the patent: **07.06.2023**
(21) Application number: 17733473.7
(22) Date of filing: 29.06.2017
(51) Int. Cl.: C12N 1/04, C12N 1/20, A61K 9/00, A61K 47/12, A61K 35/74

(54) **PRESERVATION OF MICROORGANISMS**
KONSERVIERUNG VON MIKROORGANISMEN
CONSERVATION DE MICRO-ORGANISMES

(30) Priority: 30.06.2016 BE 201605538
(43) Date of publication of application: 08.05.2019
(73) Proprietor: YUN NV, 2630 Aartselaar (BE); Universiteit Antwerpen, 2000 Antwerpen (BE)
(72) Inventor: KIEKENS, Filip, 2610 Wilrijk (BE); HENKENS, Tim, 2610 Wilrijk (BE); LEBEER, Sarah, 2020 Antwerpen (BE); CLAES, Ingmar, 2020 Antwerpen (BE)
(74) Representative: Arnold & Siedsma
(86) International application number: PCT/EP2017/066176
(87) International publication number: WO 2018/002248

(56) References cited:
- WO-A1-2008/016214
- US-A1- 2003 152 629
- US-A1- 2012 263 826
- ANTUNES A E C ET AL: "Acerola nectar with added microencapsulated probiotic", LWT- FOOD SCIENCE AND TECHNOLOGY, ACADEMIC PRESS, UNITED KINGDOM, vol. 54, no. 1, 29 April 2013 (2013-04-29) , pages 125-131, XP028575692, ISSN: 0023-6438, DOI: 10.1016/J.LWT.2013.04.018 cited in the application

## Description

### FIELD OF THE INVENTION

The present invention relates to the field of preservation of microorganisms, and in particular provides a 2-compartment system comprising a first compartment, comprising microcapsules, comprising viable microorganisms in a non-aqueous composition in the core of said microcapsules, and a second aqueous organic acid-containing compartment. It further provides methods for preserving microorganisms, on the basis of such systems, as well as uses of such systems for preserving microorganisms.

### BACKGROUND TO THE INVENTION

The use of beneficial microorganism as a probiotic in medicine has expanded over the last couple of decades. To ensure the stability of such probiotics, the bacteria need to be dried (e.g. freeze-dried or spray-dried) to obtain a metabolically inactive condition. As such they can survive for decades under the correct storage conditions (e.g. moisture, temperature,...). By the introduction of water, the bacterial powder will be reactivated and the bacteria can exert their function. Up until now, most probiotic formulations have been used for gastro-intestinal applications. Most common formulations are capsules and tablets that can easily be stored in the absence of water and that can be sealed under inert gasses such as Nitrogen or carbondioxide to set a good relative humidity for maximum stability.

On the other hand, the use of probiotics in topical (or other types of aqueous) formulations could have a huge potential as well. One option might be to formulate these in anhydrous substances such as ointments or oleogels, however patients don't generally accept those formulations. More accepted formulations are those in the form of gels/creams/foams/lotions. However, such topical formulations inherently contain a high degree of water, i.e. in order to be suitably formulated into a gel, cream, foam, lotion, ointment,.... Evidently, the presence of such high degrees of water in these formulations, poses a problem for the storage of probiotics in their metabolically inactive condition.

A second problem occurring in such aqueous (e.g. topical) formulations, is that these generally contain agents, which are not compatible with the survival of microorganisms; such as preservatives, surfactants, emulsifiers,... in order to protect such formulations against the growth of unwanted microorganisms as well as for forming stable emulsions. However, these agents of course will also form a major problem in the formulation of beneficial microorganisms.

Hence, it was an object of the present invention to provide a system allowing for long-term storage of microorganisms, which does not substantially harm such microorganisms upon use thereof. It was surprisingly found that a 2-compartment system comprising a first compartment comprising microcapsules comprising a water-insoluble and water-impermeable shell, and microorganisms contained in a non-aqueous composition in the core of said microcapsules; and a second compartment containing (or consisting of) an aqueous composition comprising one or more organic acids (having a pH of less than 7.0 and being free of buffering agents), provides a solution to the above mentioned problems. In particular, it was found that such system allows for long-term storage of the microorganisms, since, during storage they are protected from being exposed to water, in being contained in a non-water comprising compartment. Subsequently, and upon combining the content of both compartments for use, it was surprisingly found that the organic acids, which serve the purpose of preservative for storage, do not immediately harm the released microorganisms, which do become activated due to the water-component in said second composition. This is in contrast to other classes of preservatives that have a very direct working mechanism on microorganisms.

In addition, while the formulations of the present invention are particularly suitable for topical applications of probiotics, the concept of the invention could also be extended to other fields in which the problem of preserving/stabilizing microorganisms in an aqueous environment occurs. Hence, by formulating them in a 2-compartment system as defined by the present invention, these problems are resolved.

While the concept of formulating microorganisms in a 2-compartment system has already been disclosed, the prior art does not provide a solution to the protection of microorganisms once the content of both compartments is combined. In particular, the aqueous component required for activating the microorganisms is often very harsh for the microorganisms, and mostly contains preservatives, surfactants,... which are not compatible with the long-term survival of such microorganisms. In contrast, due to the selection of organic acids as preservative, we found that there was no need to include further preservatives, surfactants, or other harmful components, while the required stability of such formulations was retained.

Antunes et al., 2013 (Food Science and Technology 54: 125-131) describes a microencapsulation process in which a bacterial suspension is immobilized in cellulose acetate together with glycerol, maltodextrin, Tween,.... During spray drying, the bacterial suspension is nebulized using a single nozzle in a heated area (100°C - 200 °C). As such the water in the suspension evaporates and a dry bacterial powder is obtained. The obtained powder resembles a matrix containing said microorganisms instead of microcapsules comprising a water-insoluble and water-impermeable shell, and microorganisms contained in a non-aqueous composition in the core of said microcapsules.

US2012263826 describes an oral solution comprising at least one watery solution and jelly-like capsules comprising alginate, whey proteins and probiotics. Such jelly-like capsules are in fact a 1-fasic gel particle (i.e. matrix comprising probiotics) and not a 2-compartment system as disclosed herein, wherein the probiotics are maintained in an anhydrous core of said capsule.

WO2008016214A1 discloses a composition comprising probiotics formulated as capsules and that the microorganism is mixed with a pharmaceutically acceptable carrier to prepare a suspension or dispersing solution, which fills capsules. This document further discloses that the capsules may have an enteric coating and, that the composition may further comprise additional components such as gluconic acid. WO2008016214A1 is however clearly built around oral pharmaceutical compositions (e.g. capsules), with an enteric coating against the high acidity of the stomach acid and this document does not demonstrate a two-compartment system containing probiotics as disclosed herein.

### SUMMARY OF THE INVENTION

In a first aspect, the present invention provides a 2-compartment system consisting of:
- a first compartment comprising microcapsules comprising a water-insoluble and water-impermeable shell, further comprising viable microorganisms in a non-aqueous composition in the core of said microcapsules; and
- a second compartment comprising an aqueous composition having a pH of less than 7.0 and comprising one or more organic acids;
wherein said second compartment is free of buffering agents.

In a particular embodiment of the present invention, said second aqueous composition has a pH of less than 5.5, preferably less than 5.0, more preferably less than 4.5.

In another particular embodiment, said one or more organic acids are selected from the list comprising benzoic acid, sorbic acid, citric acid, acetic acid, lactic acid, oxalic acid, formic acid, dehydroacetic acid, fumaric acid, anisic acid, gluconic acid, malic acid, succinic acid, tartaric acid, phosphoric acid and propionic acid and derivatives thereof.

In yet a further embodiment, said viable microorganisms are probiotic microorganisms, more preferably selected from the list comprising *Lactobacillus pentosus, Lactobacillus rhamnosus,* and *Lactobacillus plantarum.*

In a particular embodiment, said first compartment is impermeable to water and oxygen.

In another particular embodiment, said water-insoluble and water-impermeable shell of the microcapsules of the present invention, is composed of alginate, xanthan gum, arabic gum, gellan gum, carrageen, gelatin, cellulose or derivatives thereof; or polymers based on agar, proteins, polyol, gelatine, PVA (polyvinyl alcohol), PLGA (Poly(lactic-co-glycolic acid), PLA (polylactic acid) and derivatives thereof, PCL, polyisohexylcyanoacrylate, acrylate derivatives, or starch, optionally in combination with chitosan, or hard fats.

In a specific embodiment, the 2-compartment system of the present invention, is in the form of a gel, cream, foam, lotion, or ointment comprising said microcapsules.

In a particular embodiment of the present invention, the non-aqueous composition is selected from the list comprising vegetable oils, mineral oils, silicon oils or hydrophilic polymers; in particular capric/caprylic triglycerides, liquid paraffin, polyethylene glycol, silicones or hard fats.

In a particular embodiment of the present invention, said one or more organic acids serve the purpose of preservative, and said composition is substantially free of further preservatives.

In a further aspect, the present invention provides a method for the preservation of viable microorganism comprising:
- providing a 2-compartment system;
- including said viable microorganisms in said first compartment comprising microcapsules comprising a water-insoluble and water-impermeable shell, and viable microorganisms contained in a non-aqueous composition in the core of said microcapsules;
- including one or more organic acids in said aqueous composition in a second compartment of said 2-compartment system in an amount sufficient to obtain a pH of less than 7.0; wherein said second compartment is free of buffering agents.

In a further aspect, the present invention provides the use of a 2-compartment system according to this invention, for the preservation of said aqueous composition, without harming said viable microorganisms.

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.
**Fig. 1****:** Stability of microorganisms in silicone; A) CF 1406, B) CF6570.
**Fig. 2****:** Stability of microorganisms in oils; A) Sunflower oil, B) Miglyol 812N.
**Fig. 3****:** Stability of microorganisms in polar media; A) PEG400, B) Glycerine.

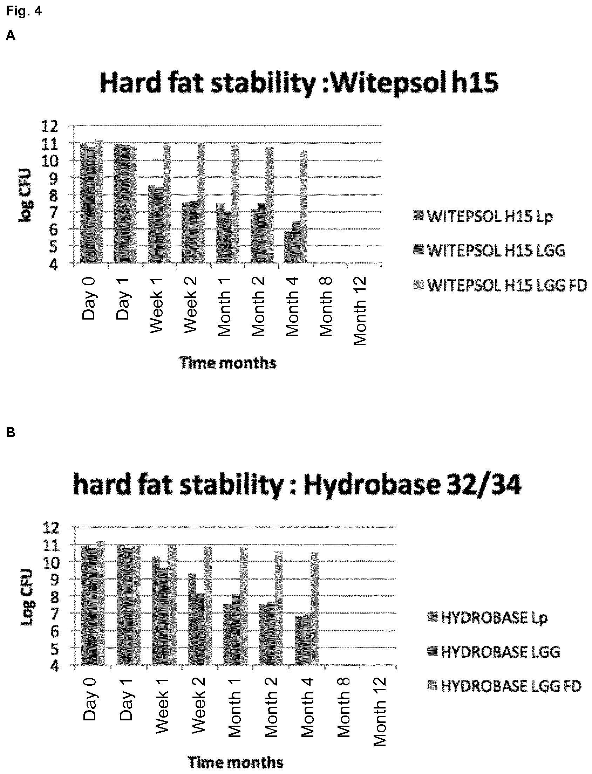

**Fig. 4****:** Stability of microorganisms in hard fats; A) Whitepsol h15, B) Hydrobase 32/34.

### DETAILED DESCRIPTION OF THE INVENTION

As already detailed herein above, in a first aspect, the present invention provides a 2-compartment system consisting of:
- a first compartment comprising microcapsules comprising a water-insoluble and water-impermeable shell, and viable microorganisms contained in a non-aqueous composition in the core of said microcapsules; and
- a second compartment comprising an aqueous composition having a pH of less than 7.0 and comprising one or more organic acids;
wherein said second compartment is free of buffering agents.

More specifically, the present invention provides a 2-compartment system as defined herein consisting of:
- microcapsules having a water-insoluble and water-impermeable shell, and viable microorganisms contained in a non-aqueous composition in the core of said shell; and
- an aqueous composition having a pH of less than 7.0 and comprising one or more organic acids;
wherein said aqueous composition is free of buffering agents.

Even more specifically, the present invention provides a 2-compartment system as defined herein consisting of:
- microcapsules that can have a water-insoluble and water-impermeable shell, and viable microorganisms contained in a non-aqueous composition in the core of said shell; and
- an aqueous composition having a pH of less than 7.0 and comprising one or more organic acids;
wherein said aqueous composition is free of buffering agents.

In the context of the present invention, the terms "water insoluble" and "water-impermeable" microcapsules are meant to be understood as water resistant. In particular, water resistant capsules can be described as not being degraded when suspended in said second aqueous compartment. Nevertheless, the microcapsules as used herein can (and usually will) degrade, e.g. lose their insolubility under certain stress conditions such as salt concentrations or mechanical shear stress, hereby releasing their active contents eg. the releasing mechanism of the capsules as described herein.

In the context of the present invention, the term 'aqueous composition' is meant to be a formulation comprising water. In particular, said formulations comprise a substantial amount of water, such as at least 10%, at least 20%, at least 30%, at least 40%, at least 50%, at least 60%, at least 70%, at least 80%, at least 90%, or at least 95% of water. Consequently, the term 'non-aqueous composition' is meant to be a formulation which do not contain substantial amounts of water, such as no more than 10%, preferably no more than 5% even more preferably no more than 2%, most preferred it contains no water at all, i.e. 0%.

In the context of the present invention the term viable microorganisms refer to microorganisms which are alive, and it is not meant to be fragments, culture supernatants, or killed forms thereof. Said viable microorganism are preferably freeze-dried in order to increase their preservation.

In the context of the present invention, the term 'organic acid' is meant to be an organic compound with acidic properties. Evidently, in the context of the present invention, any suitable organic acid, can be used in as far as it can act as a preservative.

It was found that these organic acids have a slow working mechanism on microorganisms and that they do not immediately harm the (probiotic) microorganisms once they come into contact with these organic acids. After longer exposure to the environment, such as the skin, the organic acids lose their activity, thereby also not harming the (probiotic) microorganisms over a longer period of time. While the invention is preferably performed using an organic acid as preservative (since these were found to lose their activity after exposure to the environment), it may also be performed by using another preservative in as far as it has a slow working mechanism, such as requires at least 24h to become active.

With respect to the use of organic acids, the formulations are free from buffering agents. The presence of a buffering agent will keep the compositions at a low pH for a longer period of time, thereby taking longer to inhibit the preservative action of the organic acids, and thus increasing the risk of harming the microorganisms once they come into contact with the organic acids. While most components will have a small buffering effect, it is desired to select the components of the compositions such that they do not substantially reduce or increase the time required for the inactivation of the organic acids.

Particularly suitable organic acids are those selected from the list comprising benzoic acid, sorbic acid, citric acid, acetic acid, lactic acid, anisic acid, oxalic acid, formic acid, dehydroacetic acid, fumaric acid, gluconic acid, malic acid, succinic acid, tartaric acid, phosphoric acid and propionic acid and derivatives thereof. More specifically said organic acids are selected from the list comprising: benzoic acid, sorbic acid, citric acid, acetic acid, lactic acid, oxalic acid, formic acid, dehydroacetic acid, fumaric acid, gluconic acid, malic acid, succinic acid, tartaric acid, phosphoric acid and propionic acid and derivatives thereof; such as sorbic acid.

In a particular embodiment of the present invention, the pH of the second aqueous composition according to the present invention is less than 5.5, preferably less than 5.0, more preferably less than 4.5, less than 4.0 or less than 3.5. The pH of the formulations is highly relevant within the context of the present invention. In general, the lower the pH, the higher its preservative effect, thereby contributing to the long-term stability of the formulations of the present invention. The desired pH is obtained by the co-formulated organic acids, which thus have the purpose of preservative agent in the formulations. Due to the presence of these organic acids, we found that there was no further need to include additional preservatives, hence, the formulation of the present invention is preferably substantially free of other preservatives than the organic acids. In addition, the composition is of course also substantially free from other components which are harmful for microorganisms such as phenoxyethanol, bronidox, isothiazolinones, & sodium laureth sulphate; which are very often used in topical formulations.

In a preferred embodiment, the viable microorganisms of the present invention are viable probiotic microorganisms.

In the context of the present invention, the term "probiotic" is mean to include viable microorganisms that provide health benefits when used in the human or veterinary field. The formulations of the present invention are highly suitable in the formulation of any known viable probiotic microorganisms, such as but not limited to Lactobacilli, more in particular *Lactobacillus pentosus, Lactobacillus rhamnosus,* and/or *Lactobacillus plantarum.* Evidently, the formulations of the present invention may comprise only one species of viable probiotic microorganisms, or combinations thereof, depending on the intended use.

In order to increase the stability of the microorganisms in the first compartment, said first compartment is preferably impermeable to water and oxygen.

In the context of the present invention, the terms 'microencapsulated' or 'microcapsules' are meant to refer to products obtained by a micro-encapsulation process. This is a process in which micro-sized (micrometer-range) particles or droplets are surrounded by a coating to give small capsules, of many useful properties. In the context of the present invention, it is used to incorporate probiotic microorganisms. In a relatively simple form, a microcapsule is a small sphere with a uniform wall around it. The material inside the microcapsule is referred to as the core, internal phase, or fill; whereas the wall is referred to as a shell, coating, or membrane.

A person skilled in the art is well aware of the fact that several methods for the manufacture of microcapsules exist, and that the present invention is not limited to either of such methods. Micro-encapsulation methods suitable within the context of the present invention include, but are not limited to pan coating, air-suspension coating, centrifugal extrusion, vibrational nozzle, spray drying, ionotropic gelation, coacervation-phase separation, interfacial polycondensation, interfacial cross-linking, in-situ polymerization, matrix polymerization.

The initial aim of the microencapsulation is the isolation of the core from its surrounding, in the present invention in particular in order to protect the encapsulated micro-organisms from the preservative action of the surrounding organic acids, and to protect them from being activated in an aqueous environment. Evidently, upon use the walls of the microcapsules need to be ruptured to free the micro-organisms and allow them to act as desired. Such rupture is preferably obtained by low pressure, friction or shear stress during use, such as upon application of the formulations to the skin or other topical areas.

In its simplest form the microcapsules of the present invention comprise a water-insoluble and water-impermeable shell with microorganisms contained in a non-aqueous composition in the core of said microcapsules. Where appropriate, however, the microcapsules may contain additional layers, such as for example an extra coating layer to increase protection of the content.

In a particular embodiment of the present invention, said microcapsules comprise a water-insoluble and water-impermeable shell; and said microorganisms are contained in a non-aqueous composition in the core of said microcapsules. The presence of water is sufficient to reactivate freeze-dried bacteria, hence the importance of formulating them in the absence of water, thereby allowing long-term storage. Said water-insoluble and water-impermeable shell may be composed of any suitable materials such as, but not limited to alginate, xanthan gum, arabic gum, gellan gum, carrageen, gelatin, cellulose or derivatives thereof; or polymers based on agar, proteins, polyol, gelatine, PVA (polyvinyl alcohol), PLGA (Poly(lactic-co-glycolic acid), PLA (polylactic acid) and derivatives thereof, PCL, polyisohexylcyanoacrylate, acrylate derivatives, or starch, or hard fats such as for example witepsol or hydrobase.

While these components may be used as such, they may also be combined with one another or may be cross linked to polymers or chitosan. Such further cross-linking may enhance the durability of the microcapsules. Furthermore, to protect the content of the microcapsules (i.e. the microorganisms) even further against water and air, the capsules may optionally be coated with a suitable coating.

As indicated herein before, the microorganisms should be stored in the absence of water in order to ensure long-term storage. Therefore, the core of the microcapsules of the present invention is preferably a water-free medium, such as but not limited to vegetable oils, mineral oils, silicon oils or hydrophilic polymers; in particular capric/caprylic triglycerides, liquid paraffin, polyethylene glycol, silicones or hard fats such as for example witepsol or hydrobase.

The presence of a preservative is of course highly important for the long-term storage of formulations, however, poses a serious problem for the formulation of microorganisms such as probiotics. As a solution thereto we have found that the microencapsulation of (probiotic) microorgansisms and the provision of such microcapsules in a formulation comprising organic acids, is sufficient to guarantee long term storage of the formulations, without hampering the activity of the microorganisms. Even, upon use of the formulation and release of the microorganisms, the latter are not hampered by the co-formulated organic acids, since these rapidly lose their activity once used, such as due to the buffering capacity of the skin. Therefore, the organic acids of the present invention are intended to serve the purpose of preservative, and the formulation is substantially free of further preservatives which may hamper the activity of the microorganisms after release from the microcapsules.

Hence, in a particular embodiment of the present invention said organic acid serves the purpose of preservative, and the formulation is substantially free of further preservatives.

The formulations of the present invention are particularly suitable for topical application, because such formulations in general contain a high degree of water. Therefore, in a particular embodiment, the aqueous formulation according to the present invention, is a topical aqueous formulation.

In the context of the present invention, the term "topical" is meant to be the local delivery at a specified location of the body, in particular the application to a particular place on or in the body. In particular, it includes the application to mucous membranes via aqueous, i.e. non-solid formulations such as creams, foams, gels, lotions or ointments, or any other type of water-containing formulation. Evidently, the term "topical" is not meant to include the delivery in the form of solid preparations such as capsules, tablets, ...

The topical probiotic formulations of the present invention, may be in any suitable form such as but not limited to a gel, cream, foam, lotion, or ointment, comprising said microcapsules.

As detailed herein above, the finding of the present invention is that the formulation of microorganisms, such that these do not come into contact with a water component in a 2-compartment system, in combination with the use of an organic acid is sufficient to allow for long-term storage of the formulation without substantially harming the (probiotic) microorganisms.

As such in a further aspect, the present invention provides a method for the preservation of viable microorganism comprising:
- providing a 2-compartment system;
- including said viable microorganisms in said first compartment comprising microcapsules comprising a water-insoluble and water-impermeable shell, and viable microorganisms contained in a non-aqueous composition in the core of said microcapsules;
- including one or more organic acids in said aqueous composition in a second compartment of said 2-compartment system in an amount sufficient to obtain a pH of less than 7.0; wherein said second compartment is free of buffering agents.

In a final aspect, the present invention provides the use of a 2-compartment system according to this invention for the preservation of the aqueous composition without damaging said viable microorganisms in said microcapsules.

The formulations of the present invention, are in particular highly suitable for topical administration, which includes direct application to the skin, or mucous membrane such as the vagina.

Suitable administration forms - which may be semi-solid or liquid, depending on the manner of administration - as well as methods and carriers, diluents and excipients for use in the preparation thereof, will be clear to the skilled person; reference is again made to for instance US-A-6,372,778, US-A-6,369,086, US-A-6,369,087 and US-A-6,372,733, as well as to the standard handbooks, such as the latest edition of Remington's Pharmaceutical Sciences.

Some preferred, but non-limiting examples of such preparations include elixirs, suspensions, emulsions, solutions, syrups, ointments, creams, lotions, which may be formulated with carriers, excipients, and diluents that are suitable per se for such formulations, such as lactose, dextrose, sucrose, sorbitol, mannitol, starches, gum acacia, alginates, tragacanth, gelatin, polyethylene glycol, cellulose, (sterile) water, methylcellulose, edible oils, vegetable oils and mineral oils or suitable mixtures thereof.

The formulations of the present invention can optionally contain other components such as drugs and/or prebiotics, for example for stimulating the growth of the microorganisms.

The formulations can optionally contain other substances that are commonly used in pharmaceutical formulations, such as, wetting agents, emulsifying and suspending agents, dispersing agents. However, it is very important that such further substances do not substantially harm the probiotic organisms during storage of the composition, nor upon use thereof.

More in particular, the compositions may be formulated in a pharmaceutical formulation comprising particles consisting of a solid dispersion of the microorganisms of the invention and one or more pharmaceutically acceptable water-soluble polymers.

The term "a solid dispersion" defines a system in a solid state (as opposed to a liquid or gaseous state) comprising at least two components, wherein one component is dispersed more or less evenly throughout the other component or components.

It may further be convenient to formulate the microorganisms in the form of microparticles which have a surface modifier adsorbed on the surface thereof in an amount sufficient to maintain an effective average particle size of about 1 - 5000 µm. Suitable surface modifiers can preferably be selected from known organic and inorganic pharmaceutical excipients. Such excipients include various polymers, low molecular weight oligomers, natural products and surfactants, such as nonionic and anionic surfactants.

The invention will now be illustrated by means of the following synthetic and biological examples, which do not limit the scope of the invention in any way.

### EXAMPLES

### Example 1: Selection of non-aqueous composition for first compartment

The goal of this example, was to select a suitable non-aqueous composition for including viable microorganisms, which allows for long-term stability and viability thereof.

### Material and Methods

### Stability study

Three strains were used in this stability study:
- Self-cultured *L. pentosus* (i.e. lac4 or Lp)
- Self-cultured LGG (i.e. lac7 or LGG)
- Purchased Freeze-dried LGG

Eight different water-free suspension media for preserving the bacteria were tested at room temperature (+/- 20 °C). The used suspension media can be subdivided in 4 types, for which in each group, 2 subtypes were compared as follows:
- Silicones:
   ∘ Cosmetic fluid 1406 (dimethiconol & dimethicone) - from Chemsil
   ∘ Cosmetic fluid 6570 (disiloxane, trisiloxane, cyclopentasiloxane & dimethicone) - from Chemsil
- Oils:
   ∘ Sunflower oil - from Everyday
   ∘ Migyol 812 N -from Hüls
- Polar Solvents:
   ∘ PEG 400 - from Roth
   ∘ Glycerine - from Fagron
- Hard Fats:
   ∘ Witepsol H15 - from VW chemicals
   ∘ Hydrobase 32/34 - from Prod'hyg laboratoires

Samples were obtained at 9 pre-defined timepoints:

| | |
|---|---|
| • | T0 = before suspension in the suspension media |
| • | T1 = 1 day after suspension |
| • | T2 = 1 week after suspension |
| • | T3 = 2 weeks after suspension |
| • | T4 = 1 monthsafter suspension |
| • | T5 = 2 months after suspension |
| • | T6 = 6 months after suspension |
| • | T7 = 12 months after suspension |
| • | T8 = 24 months |

All used materials were sterilized before use by autoclaving. All used components, ingredients,... were autoclaved if possible, and otherwise filtered in sterile circumstances.

### T0 CFU determination

Lac 4 & Lac 7 were cultured in liquid MRS (de MAN, Rogosa & Sharpe) medium (37 °C) until fully grown. The medium was centrifuged for 10 minutes at 2780 G. The supernatant was disposed and the bacterial pellet was further used.

100 mg was weighed and diluted with physiological water (0.85% NaCl) to obtain a total volume of 10 ml. From the three strains (lac 4, lac 7 and LGG), dilution series were made (10-fold serial dilutions) and plate counting happened according to the spread plating method (Derived from Pharmacopeia methods 2.6.12 and 2.6.13. Measurements were repeated in triplicate. Results are expressed as cfu/gram powder.

### Suspension of the bacteria in the suspension media

With the obtained bacterial masses, a homogenous 1/10 dilution (m/m %) was made with the different anhydrous suspension media. 1 gram of said suspension was weighed in a falcon tube and sealed in an aluminum bag (RH = 20%). Each sample contained 100 mg bacteria for further testing purposes. Aluminum bags were opened at certain time intervals for viability testing.

### Testing after storage time

The sealed bags were opened at certain time points for stability testing. The falcon tube containing 1 gram of anhydrous substance was further processed by adding 1 gram of an emulsifier mixture (consisting of polysorbate 80 & sorbitansesquioleate) and 8 gram of physiological water. This formed an emulsion through which the bacteria could get into contact with water and were reactivated. Further sample preparation and plate counting happened as described above and was derived from pharmacopeia method 2.6.12: Microbiological examination of non-sterile products: microbial enumeration tests and 2.6.13: microbiological examination of non-sterile products: test for specified micro-organisms.

### Results and discussion

The stability of the freeze dried powder was far more superior, as expected, compared to that of the 'as such' strains. After just 1 month, there is on average a 3 log reduction whilst there is no reduction detectable for the freeze dried strain. Hereby it is proven that it is important to start formulating with a properly freeze dried (or other drying method) powder to ensure a stable formulation of viable micro-organisms over time.

Therefore, only the results of the freeze dried LGG strain will be further discussed.

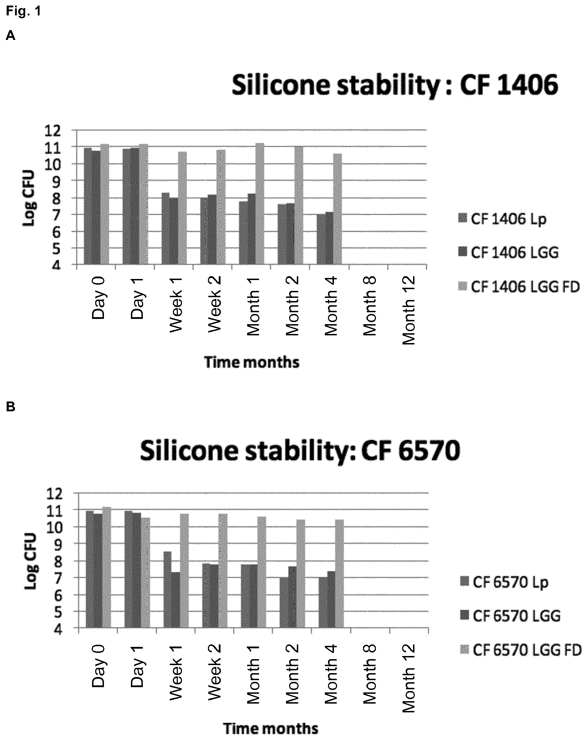

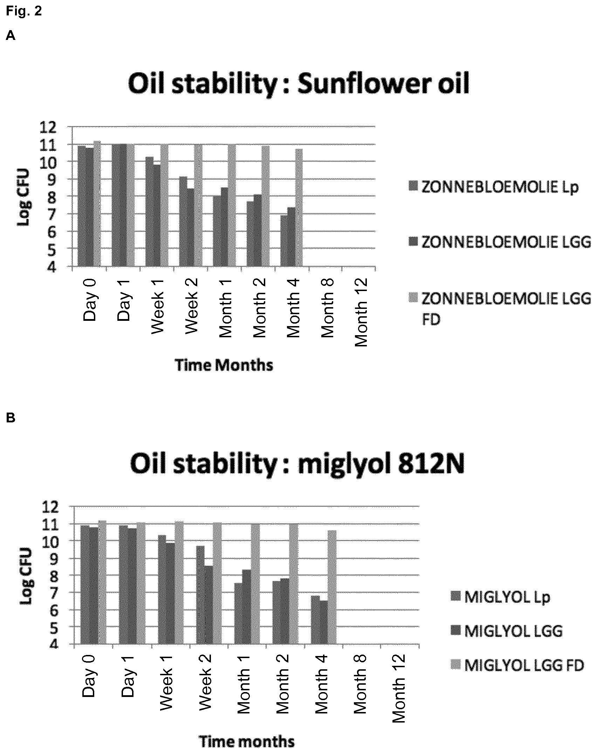

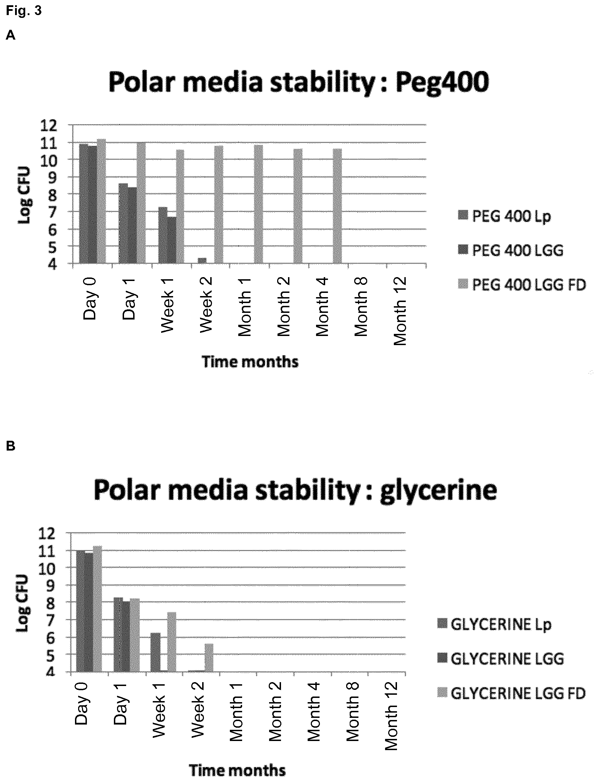

The stability results were comparable for the silicon mixtures (fig. 1), oils (fig. 2) and hard fats (fig. 3). Of the hydrophilic media (fig. 3) only PEG 400 seems suitable as a suspending medium, as opposed to glycerol, that negatively influences the stability of FD IGG.

There's an average of 1 log reduction after 1 year stability for the different anhydrous media. This leaves enough viable micro-organism to be applied topically and exert a beneficial effect on the skin.

Hereby it is proven that working with a properly freeze dried powder, suspended in anhydrous media, protected from light, moisture & preservatives, it is possible to obtain a phase/compartment with stable probiotics. This compartment can either be in a 2 phase-system, where there is in situ mixing of the aqueous and anhydrous phase or as capsules suspended in a formulation.

### Example 2: Selection of aqueous composition for second compartment

The goal is to make a topical formulation with viable micro-organisms that can be applied to the skin where they will exert a beneficial effect. A topical formulation, such as a cream, typically contains oil, water, preservatives & emulsifiers. The presence of emulsifiers & preservatives might negatively influence the stability of present freeze dried micro-organisms. A way to guarantee a certain shelf life is to suspend freeze dried probiotics in an anhydrous medium (see also example 1). This anhydrous suspension will provide a good stability for the freeze dried micro-organisms. To prevent a negative influence of other components in the topical formulation, said anhydrous suspension is best separated from the rest of the formulation, either through physical phase separation or by encapsulating said anhydrous suspension. The capsules can then be suspended in a topical formulation.

As long as the phases are separated, a long lasting topical product with viable micro-organisms can be obtained. A problem might occur when the 2 phases are mixed, before application onto the skin. The freeze dried probiotics will be activated through water uptake from the formulation, but also encounter present emulsifiers & preservatives, killing off the activated micro-organisms, and preventing them from exerting a beneficial effect to the skin. This experiment is a screening to determine the short term effect (10 minutes -> 1 day) of different formulations on the survival of freeze dried probiotics.

### 1. METHOD

### 1.1 Experiment A

This experiment is a simulation where viable, freeze dried micro-organisms are contained stable in an anhydrous suspension and are then mixed with a formulation containing different preservatives and emulsifiers. Three standard TMF preparations are incubated with 10 % (m/m) freeze dried LGG. The TMF formulations are widely used (Belgium) and regarded as safe and stable formulations for the topical delivery of drugs. A sample from the formulation is taken after 10 minutes and after 24 hours to determine the viability, and thus the short term antibacterial effect of said formulations. Application of topical probiotics typically occurs every day. Therefore, no samples after 24 hours were taken. Sample preparation and CFU determination (plate counting) happened according to the spread plating method (Derived from Pharmacopeia methods 2.6.12 and 2.6.13. Measurements were repeated in triplicate. Results are expressed as cfu/gram powder.

The composition of the tested creams is as follows:

| Cream A : buffered cetomacrogol cream **- WITH** organic acid | |
|---|---|
| Ingredient | % |
| Cetostearylalcohol | 7,2 |
| Cetomacrogol | 1,8 |
| white vaseline | 15 |
| liquid paraffinum | 6 |
| Potassium sorbate | 0,27 |
| Sodiumdihydrogenphosphate | 0,3 |
| NaOH/ Phosphoric acid qs pH5 | qs |
| aqua purificata | ad 100 |

| Cream B: Anionic hydrophilic cream - **WITHOUT** organic acid | |
|---|---|
| Ingredient | % |
| Cetostearylalcohol | 15 |
| glycerol | 5 |
| Sodium lauryl sulphate | 1,5 |
| methylparabens | 0,06 |
| Propylbarabens | 0,03 |
| aqua purificata | ad 100 |

| Cream C : AVA cream - **WITH** organic acid | |
|---|---|
| Ingredient | % |
| White vaseline | 54 |
| Sorbitansesquioleate | 6 |
| sorbic acid | 0,27 |
| aqua purificata | ad 100 |

### 1.2 Experiment B

To extend the screening platform of experiment A, Experiment A was repeated with commercially available formulations containing different preservatives and emulsifiers. The conduct of the experiment is the same as described in 2.1. Cream A from experiment A was taken as reference. Other pharmaceutical cream bases (1-5) were screened as well as compared to commercially available cosmetic formulations (6-11), as detailed in the following table:

| |
|---|
| 1)Buffered cetomacrogol cream |
| 2)Lanette cream |
| 3)Carbomeergel |
| 4)Nourivan |
| 5)Pentravan |
| 6)Commercial cosmetic composition 1 (CCC1) |
| 7)CCC2 |
| 8)CCC3 |
| 9)CCC4 |
| 10)CCC5 |
| 11)CCC6 |

### 2. RESULTS

### 2.1 experiment A

| Survival | 10 minutes | 24 hours |
|---|---|---|
| Blanc sample | 2.0E+11 | 9,5E+10 |
| Cream A | 1,7E+11 | 7,9E+10 |
| Cream B | 1,0E+11 | 3,1E+08 |
| Cream C | 1,2E+11 | 3,2E+10 |

Survival of freeze dried LGG seems to be only minimally affected after being suspended for 10 minutes in the above formulations. Nearly no decrease is noticeable for cream A after 24 hours. A slight decrease (± 0.5 log) was noted for cream C and a big decrease (± 3 log) for cream B. Both cream A & C contain the organic acid Sorbic acid as preservative, as opposed to cream B that contains parabens as preservative agent and Sodium lauryl sulphate (an anionic emulsifier with known antimicrobial properties).

All in all it can be concluded that the organic acids have a slow working mechanism (time required to be effective is 1-3 days (data not shown)) and might thus be regarded as a safe preservative for use in topical formulations in combination with freeze dried micro-organisms. Formulation A is suggested for further trials regarding the combination of freeze dried probiotics in a topical formulation.

### 2.2 Experiment B

| Survival | 10 minutes | 24 hours |
|---|---|---|
| Blanc sample | 9,4E+10 | |
| 1)Buffered cetomacrogol cream | 1,2E+11 | 7,8E+10 |
| 2)Lanette cream | 2,8E+10 | 0,0E+00 |
| 3)Carbomeergel | 2,3E+10 | 5,1E+09 |
| 4)Nourivan | 4,9E+10 | 1,8E+10 |
| 5)Pentravan | 4,0E+10 | 1,6E+08 |
| 6) CCC1 | 0 | 0 |
| 7) CCC2 | 0 | 0 |
| 8) CCC3 | 0 | 0 |
| 9) CCC4 | 0 | 0 |
| 10) CCC5 | 0 | 0 |
| 11) CCC6 | 0 | 0 |

| | | |
|---|---|---|
| *The used method had a limit of detection of 4 log reduction. A CFU count of 0 means less than 1,0E+07 CFU/gram powder was detected. | | |

At first notice, the pharmaceutical cream bases (1-5) seem to be less antibacterial on short term than the cosmetic formulations (6-10) tested. The cosmetic formulations are extremely antibacterial (> 4 log reduction after 10 minutes) and their ingredients (emulsifiers & preservatives) are deemed not suitable for formulation purposes. The main problem ingredients have been identified as phenoxyethanol, bronidox, isothiazolinones, & sodium laureth sulphate. Other ingredients with antibacterial properties are present as well but are of lesser importance.

The best formulation (after 24 hours) is again the buffered cetomacrogol cream, with sorbic acid as only preservative (± 0.15 log reduction after 24 hours). The formulation with the 2^{nd} best survival is Nourivan pharmaceutical base, containing only sorbic acid as preservative. The 3d best formulation is carbomeergel containing parabens and the fourth best formulation is the pentravan cream containing sorbic acid & benzoic acid as preservatives. Lanette cream contains parabens & sorbic acid as preservative.

It is clear that organic acids (and parabens) are slow working preservatives as opposed to those commonly found in cosmetic formulations, and do not substantially harm the probiotics after application to the skin. Formulations 1, 4 & 5, containing only organic acid preservatives, show the least reduction after 10 minutes. While parabens, may also be suitable for use as a preservatives, they are known allergenic agents and are thus preferably not used in the cosmetic industry. Therefore, it is concluded that the organic acid preservatives are a suitable option to co-formulate with probiotics.

### Example 3: Stability Assays

Our capsules form a homogeneous wall (shell) around the inner core that contains the probiotic bacteria. This shell protects the bacteria and make sure they are not immediately released in the aqueous environment once our capsules are suspended.

We can provide good stability at both refrigerated and room temperatures as evident from the below table:

| Capsules in creme (cfu/g creme) | | | | | | | |
|---|---|---|---|---|---|---|---|
| Time | 4 °C | A | B | c | | AVG | stdev |
| 0w | | | | | | 4,7E+09 | 4,2E+08 |
| 2w | | 3,7E+09 | 3,8E+09 | 3,7E+09 | | 3,7E+09 | 4,7E+07 |
| 1m | | 3,0E+09 | 2,9E+09 | 2,9E+09 | | 2,9E+09 | 4,7E+07 |
| 2m | | 9,3E+08 | 1,9E+09 | 8,5E+08 | | 1,2E+09 | 4,8E+08 |
| 3m | | 5,0E+08 | 5,7E+08 | 4,5E+08 | | 5,1E+08 | 4,9E+07 |
| 6m | | 3,4E+06 | 5,5E+07 | 3,6E+06 | | 2,1E+07 | 2,4E+07 |
| | | | | | | | |

| Time | 25 °C | A | B | c | | AVG | stdev |
|---|---|---|---|---|---|---|---|
| 0w | | | | | | 4,7E+09 | 4,2E+08 |
| 2w | | 2,0E+09 | 2,6E+09 | 1,4E+09 | | 2,0E+09 | 4,9E+08 |
| 1m | | 7,5E+08 | 2,4E+09 | 1,9E+09 | | 1,7E+09 | 6,9E+08 |
| 2m | | 1,0E+08 | 5,4E+08 | 1,4E+08 | | 2,6E+08 | 2,0E+08 |
| 3m | | 1,8E+06 | 6,0E+07 | 2,2E+07 | | 2,8E+07 | 2,4E+07 |
| 6m | | 3,3E+06 | 7,5E+06 | 1,8E+06 | | 4,2E+06 | 2,4E+06 |

### Example 4: Viability assays in creams containing organic acids as preservative

To object of this example was to determine the efficacy of the probiotic capsules, when suspended in an Oil/water cream in the presence of sorbic acid preservative in different concentrations. A second goal was to prove that the chosen preservatives are suitable for protecting the cream. This was proven by conducting a challenge test on the cream (without the probiotic capsules) with the different preservative concentrations, according to the European Pharmacopeia (5.1.3).

### Material & Methods:

### Used compositions

A basic cream without preservatives/perfume was used as basis for further tests. The preservative tested was Sorbic acid (2,4 hexadienoic acid), an organic acid with pKa 4.76 at 25 °C at different concentrations. The pH of the cream for all the tested conditions was adjusted to 4.5 using HCl/NaOH.

### Challenge tests & efficacy of organic acid preservative concentrations

Challenge tests are used to determine the efficacy of the antimicrobial conservation system for dermatological products. The goal is to increase the safety and durability of the product. Sorbic acid was tested in concentrations : 0.5% - 0.3% - 0.1% - 0% (Control).

The challenged organisms were *E. coli (DSM 1576), Ps. Aeruginosa (DSM 1128), C. albicans(DSM 1386), St. aureus(DSM 799), As. Niger (DSM 1988).*

Addition of the challenged organisms (in 100 gram product) happens at 10⁶ CFU/ML. Sampling happens after 6 hours, 24 hours, 7-14 & 28 days. A challenge test is successful if at least a 3 log reduction is visible after 7 days for the bacteria and a 2 log reduction for the yeasts/molds after 14 days - 3 log after 4 weeks. Results shown are after 4 weeks of sampling.

### Viability of probiotic capsules in cream base with different preservatives

The probiotic capsules are suspended 15 % (m/) in the cream base with the different preservative concentrations. To simulate release of the probiotics *in vivo,* the use of a Citric acid buffer was used *in vitro.* The citric acid will dissolve the membrane of the capsule and hereby release the probiotics inside the capsule. CA buffer was made by dissolving 31.2g Na2HPo4 & 1.25 g Citric acid in 971.54 g Sterile water. The Final solution is isotonic with a pH of 7.4t 0.2.

The sample creams are sampled in duplo after which it is dissolved in CA-buffer. A further 1/10 dilution series is made and 0.1 ml is spread on MRS agar plates using the 'copacabana spread plating method' with 5 glass beads diameter 4mm. Plating happens in duplo and results (after 3 days incubation 37 °C) are calculated as CFU/ gram cream. Storage happens at 4°C & 25 °C. Sampling happens after 1-2-3 months. Results shown are the stability of the capsules after 3 months during cold storage.

### Results

### Challenge test results

Challenge test results on base cream (excluding probiotic capsules) after 4 weeks.

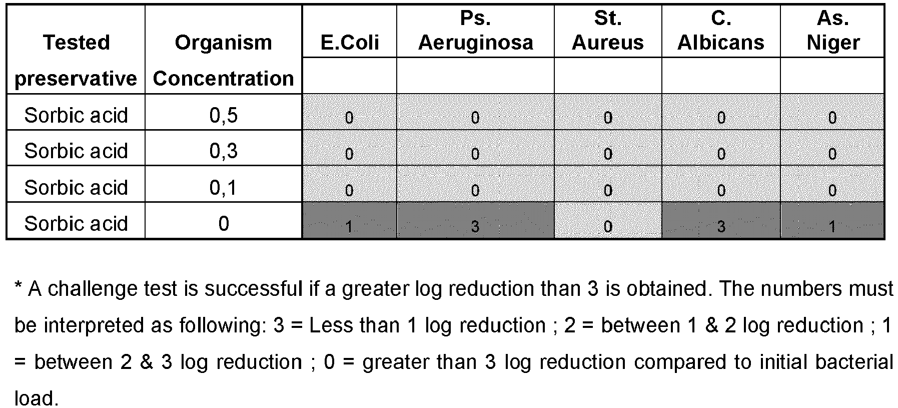

### Viability of probiotic capsules in cream with different concentrations of sorbic acid

Survival of probiotic capsules after 3 months in basic cream in presence of different sorbic acid preservative concentrations.

| | Concentration % | CFU/g | Stdev | % survival |
|---|---|---|---|---|
| Blanc | 0 | 9,E+07 | 1,E+07 | 100 |
| Sorbic acid | 0,3 | 7,E+07 | 1,E+07 | 76 |
| Sorbic acid | 0,1 | 7,E+07 | 2,E+07 | 79 |
| Sorbic acid | 0,03 | 6,E+07 | 4,E+07 | 68 |

### Conclusion

Organic acids (such as sorbic acid) are a valuable preservative for dermatological formulations. Sorbic acid has a good preservative effect in concentrations as low as 0.1%. No addition of Sorbic acid resulted in a failure of the challenge test as it seemed to be unable to kill of all the tested micro-organisms in the foreseen timeframe. Therefore the use of organic acids as sole preservative in a formulation seems to be suitable in preventing spoilage by either bacteria, yeast or molds, when present in a suitable concentration.

Suspending the probiotic capsules (15% m/m) in the same base cream that was used for the challenge tests, revealed that the probiotic capsules were not affected by either of the different preservative concentrations. The bacterial CFU load of the cream containing the different preservatives varied no more than 32% (less than 0.3 log reduction) compared to the blanc. This is well within the deviation and is no significant difference.

This implies that the probiotic capsules offer a good protection against the organic acids tested in this experiment in this cream formulation, whilst at the same time the preservatives are able to prevent growth of exogenous organisms in the cream.

## Claims

1. A 2-compartment system consisting of:
- a first compartment comprising microcapsules comprising a water-insoluble and water-impermeable shell, and viable microorganisms contained in a non-aqueous composition in the core
of said microcapsules; and
- a second compartment comprising an aqueous composition having a pH of less than 7.0 and comprising one or more organic acids;
wherein said second compartment is free of buffering agents.

2. The 2-compartment system according to claim 1, wherein said aqueous composition of the second compartment has a pH of less than 5.5, preferably less than 5.0, more preferably less than 4.5.

3. The 2-compartment system according to anyone of claims 1 or 2; wherein said one or more organic acids are selected from the list comprising benzoic acid, sorbic acid, citric acid, acetic acid, lactic acid, anisic acid, oxalic acid, formic acid, dehydroacetic acid, fumaric acid, gluconic acid, malic acid, succinic acid, tartaric acid, phosphoric acid and propionic acid and derivatives thereof.

4. The 2-compartment system according to anyone of claims 1 to 3; wherein said viable microorganisms are viable probiotic microorganisms, more preferably selected from the list comprising *Lactobacillus pentosus, Lactobacillus rhamnosus,* and *Lactobacillus plantarum.*

5. The 2-compartment system according to anyone of claims 1 to 4; wherein said micro- organism containing first compartment is impermeable to water and oxygen.

6. The 2-compartment system according to anyone of claims 1 to 5; wherein said water-insoluble and water-impermeable shell is composed of alginate, xanthan gum, arabic gum, 30 gellan gum, carrageen, gelatin, cellulose or derivatives thereof; or polymers based on agar, proteins, polyol, gelatine, PVA (polyvinyl alcohol), PLGA (Poly(lactic-co-glycolic acid), PLA (polylactic acid) and derivatives thereof, PCL, polyisohexylcyanoacrylate, acrylate derivatives, or starch, optionally in combination with chitosan; or hard fats.

7. The 2-compartment system according to anyone of claims 1 to 6; which is in the form of a gel, cream, foam, lotion, or ointment comprising said microcapsules.

8. The 2-compartment system according to anyone of claims 1 to 7; wherein said non- aqueous composition is selected from the list comprising vegetable oils, mineral oils, silicon oils or hydrophilic polymers; in particular capric/caprylic triglycerides, liquid paraffin, polyethylene glycol, silicones or hard fats.

9. The 2-compartment system according to anyone of claims 1 to 8; wherein said organic acid serves the purpose of preservative, and wherein the composition is substantially free of further preservatives.

10. A method for the preservation of viable microorganisms comprising:
- providing a 2-compartment system according to claim 1
- including said viable microorganisms in said first compartment comprising microcapsules comprising a water-insoluble and water-impermeable shell, and viable microorganisms contained in a non-aqueous composition in the core of said microcapsules;
- including one or more organic acids in said aqueous composition in a second compartment of said 2-compartment system in an amount sufficient to obtain a pH of less than 7.0; wherein said second compartment is free of buffering agents.

11. Use of a 2-compartment system according to claim 1, for the preservation of the aqueous composition without damaging said viable microorganisms in said microcapsules.

## Patentansprüche

1. Ein 2-Kammer-System bestehend aus:
- einer ersten Kammer, aufweisend Mikrokapseln, aufweisend eine wasserunlösliche und wasserundurchlässige Hülle, und lebensfähige Mikroorganismen, die in einer nicht-wässrigen Zusammensetzung im Kern der Mikrokapseln enthalten sind; und
- eine zweite Kammer, aufweisend eine wässrige Zusammensetzung mit einem pH-Wert von weniger als 7,0 und aufweisend eine oder mehrere organische Säuren;
wobei die zweite Kammer frei von Puffersubstanzen ist.

2. Das 2-Kammer-System nach Anspruch 1, wobei die wässrige Zusammensetzung der zweiten Kammer einen pH-Wert von weniger als 5,5, bevorzugt weniger als 5,0, besonders bevorzugt weniger als 4,5 aufweist.

3. Das 2-Kammer-System nach einem der Ansprüche 1 oder 2, wobei die eine oder mehreren organischen Säuren aus der Liste, aufweisend Benzoesäure, Sorbinsäure, Zitronensäure, Essigsäure, Milchsäure, Anissäure, Oxalsäure, Ameisensäure, Dehydroessigsäure, Fumarsäure, Gluconsäure, Apfelsäure, Bernsteinsäure, Weinsäure, Phosphorsäure und Propionsäure und Derivate davon, ausgewählt sind.

4. Das 2-Kammer-System nach einem der Ansprüche 1 bis 3, wobei die lebensfähigen Mikroorganismen lebensfähige probiotische Mikroorganismen sind, die bevorzugt aus der Liste, aufweisend Lactobacillus Pentosus, Lactobacillus Rhamnosus und Lactobacillus Plantarum, ausgewählt sind.

5. Das 2-Kammer-System nach einem der Ansprüche 1 bis 4, wobei die Mikroorganismen enthaltende erste Kammer für Wasser und Sauerstoff undurchlässig ist.

6. Das 2-Kammer-System nach einem der Ansprüche 1 bis 5, wobei die wasserunlösliche und wasserundurchlässige Hülle aus Alginat, Xanthan, Gummi Arabicum, 30 Gellan, Carrageen, Gelatine, Cellulose oder Derivaten davon; oder aus Polymeren auf der Basis von Agar, Proteinen, Polyol, Gelatine, PVA (Polyvinylalkohol), PLGA (Poly(lactid-co-glycolid)), PLA (Polylactylchlorid) und deren Derivate, PCL, Polyisohexylcyanoacrylat, Acrylatderivate oder Stärke, gegebenenfalls in Kombination mit Chitosan; oder Hartfette.

7. Das 2-Kammer-System nach einem der Ansprüche 1 bis 6, das in Form eines Gels, einer Creme, eines Schaums, einer Lotion oder einer Salbe ist, aufweisend die Mikrokapseln.

8. Das 2-Kammer-System nach einem der Ansprüche 1 bis 7, wobei die nichtwässrige Zusammensetzung aus der Liste, aufweisend pflanzliche Öle, Mineralöle, Silikonöle oder hydrophile Polymere, insbesondere Capric/Capryl-Triglyceride, flüssiges Paraffin, Polyethylenglykol, Silikone oder Hartfette, ausgewählt ist.

9. Das 2-Kammer-System nach einem der Ansprüche 1 bis 8, wobei die organische Säure als Konservierungsmittel vorgesehen ist und die Zusammensetzung im Wesentlichen frei von weiteren Konservierungsmitteln ist.

10. Ein Verfahren zur Konservierung von lebensfähigen Mikroorganismen, umfassend:
- Bereitstellen eines 2-Kammer-Systems nach Anspruch 1
- Einschließen der lebensfähigen Mikroorganismen in die erste Kammer, aufweisend Mikrokapseln, die eine wasserunlösliche und wasserundurchlässige Hülle aufweisen, und lebensfähige Mikroorganismen, die in einer nicht-wässrigen Zusammensetzung im Kern der Mikrokapseln enthalten sind;
- Einschließen einer oder mehrerer organischer Säuren in die wässrige Zusammensetzung in einer zweiten Kammer des 2-Kammer-Systems in einer Menge, die ausreicht, um einen pH-Wert von weniger als 7,0 zu erhalten;
wobei die zweite Kammer frei von Puffermitteln ist.

11. Eine Verwendung eines 2-Kammer-Systems nach Anspruch 1 zur Konservierung der wässrigen Zusammensetzung ohne Schädigung der lebensfähigen Mikroorganismen in den Mikrokapseln.

## Revendications

1. Système à deux compartiments constitué de :
- un premier compartiment comprenant des microcapsules comprenant une enveloppe insoluble dans l'eau et imperméable à l'eau, et des micro-organismes viables contenus dans une composition non aqueuse dans le noyau desdites microcapsules ; et
- un second compartiment comprenant une composition aqueuse ayant un pH inférieur à 7,0 et comprenant un ou plusieurs acides organiques ;
dans lequel ledit second compartiment est exempt d'agents tampons.

2. Système à deux compartiments selon la revendication 1, dans lequel ladite composition aqueuse du second compartiment a un pH inférieur à 5,5, de préférence inférieur à 5,0, de manière davantage préférée inférieur à 4,5.

3. Système à deux compartiments selon l'une quelconque des revendications 1 ou 2 ; dans lequel lesdits un ou plusieurs acides organiques sont choisis dans la liste comprenant l'acide benzoïque, l'acide sorbique, l'acide citrique, l'acide acétique, l'acide lactique, l'acide anisique, l'acide oxalique, l'acide formique, l'acide déshydroacétique, l'acide fumarique, l'acide gluconique, l'acide malique, l'acide succinique, l'acide tartrique, l'acide phosphorique et l'acide propionique et les dérivés de ceux-ci.

4. Système à deux compartiments selon l'une quelconque des revendications 1 à 3 ; dans lequel lesdits micro-organismes viables sont des micro-organismes probiotiques viables, de manière davantage préférée choisis dans la liste comprenant *Lactobacillus pentosus, Lactobacillus rhamnosus* et *Lactobacillus plantarum.*

5. Système à deux compartiments selon l'une quelconque des revendications 1 à 4 ; dans lequel ledit premier compartiment contenant des micro-organismes est imperméable à l'eau et à l'oxygène.

6. Système à deux compartiments selon l'une quelconque des revendications 1 à 5 ; dans lequel ladite enveloppe insoluble dans l'eau et imperméable à l'eau est composée d'alginate, de gomme de xanthane, de gomme arabique, de gomme gellane 30, de carraghénane, de gélatine, de cellulose ou de dérivés de ceux-ci ; ou de polymères à base d'agar agar, de protéines, de polyol, de gélatine, de PVA (alcool polyvinylique), de PLGA (poly(acide lactique-co-glycolique), de PLA (acide polylactique) et de dérivés de celui-ci, de PCL, de polyisohexylcyanoacrylate, de dérivés d'acrylate, ou d'amidon, optionnellement en combinaison avec du chitosane ; ou de graisses dures.

7. Système à deux compartiments selon l'une quelconque des revendications 1 à 6 ; qui est sous la forme d'un gel, d'une crème, d'une mousse, d'une lotion ou d'un onguent comprenant lesdites microcapsules.

8. Système à deux compartiments selon l'une quelconque des revendications 1 à 7 ; dans lequel ladite composition non aqueuse est choisie dans la liste comprenant les huiles végétales, les huiles minérales, les huiles de silicium ou les polymères hydrophiles ; en particulier les triglycérides capriques/capryliques, la paraffine liquide, le polyéthylène glycol, les silicones ou les graisses dures.

9. Système à deux compartiments selon l'une quelconque des revendications 1 à 8 ; dans lequel ledit acide organique sert de conservateur, et dans lequel la composition est sensiblement exempte de conservateurs supplémentaires.

10. Procédé de conservation de micro-organismes viables comprenant :
- la fourniture d'un système à deux compartiments selon la revendication 1
- l'inclusion desdits micro-organismes viables dans ledit premier compartiment comprenant des microcapsules comprenant une enveloppe insoluble dans l'eau et imperméable à l'eau, et des micro-organismes viables contenus dans une composition non aqueuse dans le noyau desdites microcapsules ;
- l'inclusion d'un ou plusieurs acides organiques dans ladite composition aqueuse dans un second compartiment dudit système à deux compartiments en une quantité suffisante pour obtenir un pH inférieur à 7,0 ; dans lequel ledit second compartiment est exempt d'agents tampons.

11. Utilisation d'un système à deux compartiments selon la revendication 1, pour la conservation de la composition aqueuse sans endommager lesdits micro-organismes viables dans lesdites microcapsules.
